(19) Europäisches Patentamt
European Patent Office
Office européen des brevets

(11) **EP 1 455 647 B1**

(12) **EUROPÄISCHE PATENTSCHRIFT**

(45) Veröffentlichungstag und Bekanntmachung des Hinweises auf die Patenterteilung:
**31.12.2008 Patentblatt 2009/01**

(21) Anmeldenummer: **02796547.4**

(22) Anmeldetag: **20.11.2002**

(51) Int Cl.:
***A61B 5/05*** (2006.01)

(86) Internationale Anmeldenummer:
**PCT/EP2002/013026**

(87) Internationale Veröffentlichungsnummer:
**WO 2003/043493 (30.05.2003 Gazette 2003/22)**

(54) **ELEKTRODE FÜR BIOMEDIZINISCHE MESSUNGEN**

ELECTRODE FOR BIOMEDICAL MEASUREMENTS

ELECTRODE CONCUE POUR DES MESURES BIOMEDICALES

(84) Benannte Vertragsstaaten:
**AT BE BG CH CY CZ DE DK EE ES FI FR GB GR IE IT LI LU MC NL PT SE SK TR**

(30) Priorität: **20.11.2001 DE 10156833**

(43) Veröffentlichungstag der Anmeldung:
**15.09.2004 Patentblatt 2004/38**

(73) Patentinhaber:
• **Böhm, Stephan, Dr.**
**20251 Hamburg (DE)**
• **Amato, Marcelo B.P.**
**05403-900 Sao Paulo (BR)**

(72) Erfinder:
• **HELZEL, Thomas**
**24568 Kaltenkirchen (DE)**
• **SUAREZ SIPMANN, Fernando**
**E-28010 Madrid E (ES)**
• **KOWALEWSKI, Gerd**
**24568 Kaltenkirchen (DE)**

(74) Vertreter: **Joppich, Martin**
**Zeppelinstrasse 71-73**
**81669 München (DE)**

(56) Entgegenhaltungen:
US-A- 3 882 846      US-A- 4 182 314
US-A- 5 709 213      US-A- 6 016 445
US-B1- 6 236 886

EP 1 455 647 B1

## Beschreibung

Die Erfindung betrifft eine Elektrode für biomedizinische Messungen, insbesondere für Messungen der elektrischen Impedanz Tomographie.

Bei der elektrischen Impedanz Tomographie zur regionalen Messung der Druck-Volumen-Verhältnisse werden eine Anzahl von Elektroden um den Brustkorb gelegt, wobei ein Wechselstrom beispielsweise im Bereich von 1 kHz bis 1 MHz bei einer Amplitude im Bereich von 1 μA bis 10 mA an jeweils benachbarte Elektroden angelegt wird. Die jeweils anderen Elektroden werden bei anliegendem Wechselstrom benutzt, um eine Impedanzmessung gegenüber einem definierten Bezugspotential durchzuführen. Sobald alle Elektroden der Reihe nach als stromführende Elektroden gedient haben, ist ein Zyklus zur Datenerfassung abgeschlossen. Um statistische Störungen zu eliminieren, werden in der Regel mehrere Datenerfassungszyklen gemittelt, um ein entsprechendes Bild zu erhalten. Die größten Impedanzänderungen im Bereich des Thorax entstehen durch das Einatmen und Ausatmen von Luft. Hierbei kann beobachtet werden, dass die von den Elektroden gemessene Impedanzänderung ein Maß für die Volumenänderung in der Lunge ist. Aufgrund einer rechnergestützten Auswertung der Signale an den Elektroden kann ein zweidimensionales oder auch dreidimensionales Abbild der Impedanzänderungen erstellt werden.

Die künstliche Beatmung der kranken Lunge, in der sich Ödeme gebildet haben, ist ein besonderes Problem, da nicht genau kontrolliert werden kann, ob die Lunge in bestimmten Teilen bereits verschlossen bzw. kollabiert ist. Dabei wurde herausgefunden, dass die Mortalitätsrate wesentlich gesenkt werden kann, wenn ein bestimmter Druck in der Lunge künstlich aufrecht erhalten wird, der gerade noch das Offenhalten sämtlicher Alveolen (Lungenbläschen) ermöglicht.

Hierzu wird in WO 00/33733 beschrieben, wie aus den gemessenen Impedanzänderungen mit der elektrischen Impedanz Tomographie das alveoläre Öffnen und das alveoläre Schließen der Lunge in Abhängigkeit des Beatmungsdrucks bestimmt werden kann. Bei dieser lebenswichtigen Anwendung müssen allerdings größere Messfehler weitestgehend ausgeschlossen werden.

Wesentliche Messfehler bei der elektrischen Impedanz Tomographie sind in den sich ändernden Impedanzen der Zuleitungen zu den Elektroden sowie in den Übergangswiderständen zwischen der Haut des Patienten und den Elektroden begründet. Da diese Störimpedanzen in Reihe zu der zu messenden Impedanz liegen, gehen die Störimpedanzen direkt als Fehler in die Messung ein.

In US 5,544,662 sind verschiedene schaltungstechnische Maßnahmen für ein Auswertegerät beschrieben, um die oben erwähnten Messfehler zu reduzieren. Die jeweiligen Elektroden sind aber nach wie vor über Zuleitungen angeschlossen, die von den Elektroden zu einem neben dem Patienten aufgestellten Auswertegerät führen, so dass durch Impedanzänderungen an den Zuleitungen weiterhin Messfehler entstehen.

In J.D. Bronzino, The Biomedical Engineering Handbook, CRC Press, 1995, Seiten 745 bis 757, sind verschiedene Arten von sogenannten Biopotential-Elektroden beschrieben, die als passive Elektroden dazu ausgelegt sind, Potentiale am Körper abzugreifen. Unter anderem wird auch erwähnt, dass als Spannungsfolger konfigurierte Operationsverstärker auf der Elektrode integriert sein können. Eine weitere Beschaltung der in der Elektrode integrierten Elektronik ist allerdings nicht erwähnt. Dies rührt vor allem daher, dass die Elektroden aus hygienischen Gründen als EinwegArtikel dienen müssen und von daher sich schon aus Kostengründen eine aufwendige Beschaltung der Elektrode verbietet.

Aufgabe der Erfindung ist es, eine Elektrode für biomedizinische Messungen und insbesondere für Messungen mit der elektrischen Impedanz Tomographie bereitzustellen, die möglichst störungsfreie Messungen bei kostengünstiger Fertigung erlaubt.

Diese Aufgabe wird durch eine Elektrode nach dem Patentanspruch 1 und einen Elektrodengürtel nach dem Patentanspruch 12 gelöst.

Die erfindungsgemäße Elektrode umfasst eine Kontaktplatte, einen Leitungstreiber, der einen hochohmigen Signaleingang und einen niederohmigen Leitungsausgang aufweist, und eine Stromquelle, die einen Stromausgang sowie einen Bezugspunkt aufweist, wobei die Kontaktplatte mit dem hochohmigen Signaleingang des Leitungstreibers und mit dem Stromausgang der Stromquelle elektrisch verbunden ist und wobei der Leitungstreiber und die Stromquelle in räumlicher Nähe zur Kontaktplatte angeordnet sind.

Die wesentliche Erkenntnis der Erfindung besteht darin, dass die Kombination eines Leitungstreibers und einer Stromquelle als integrierte Elektronik auf oder in der Nähe der Kontaktplatte bereits eine wesentliche Genauigkeitssteigerung gegenüber bekannten Elektroden bei weiterhin geringen Fertigungskosten ermöglicht. Damit ist die Fertigung einer integrierten Elektrode als Einwegteil zu vertretbaren Kosten möglich.

Die erfindungsgemäße Elektrode eignet sich für alle biomedizinischen Messungen, bei denen ein Strom über die Kontaktplatte eingeprägt und ein Potential über die Kontaktplatte gemessen wird, wie dies beispielsweise bei der elektrischen Impedanz Tomographie der Fall ist. Die Erfindung beruht dabei auf der Erkenntnis, dass sowohl die Stromzuführung an die Kontaktplatte, als auch die Messwerterfassung von der Kontaktplatte in räumlicher Nähe zur Kontaktplatte erfolgen muss. Dies wird durch einen hohen Innenwiderstand der Stromquelle und einen hohen Eingangswiderstand des Leitungstreibers erreicht, so dass die Störungen auf den Leitungen für die äußere Stromzufuhr und für die Weiterleitung der Messsignale vernachlässigt werden können. Die räumliche Nähe der Stromquelle und des Leitungstreibers zu der Kontaktplatte definiert sich über die Ausdehnung der Kontaktplatte. Die räumliche Nähe im Sinne der Erfindung ist zumindest dann nicht mehr gegeben, wenn das 10-fache der räumlichen Ausdehnung der Kontaktplatte überschritten wird.

Nach einer bevorzugten Ausführungsform ist vorgesehen, dass die elektrischen Verbindungen zwischen der Kontaktplatte, der Stromquelle sowie dem Leitungstreiber derart ausgestaltet sind, dass sich eine möglichst niedrige Streukapazität zwischen Kontaktplatte und Bezugspunkt ergibt. Besonders vorteilhaft ist es hierbei, wenn der Leitungstreiber und die Stromquelle sich als integrierte Schaltung auf der Kontaktplatte befinden, da hiermit die Zuleitungslängen zwischen Kontaktplatte sowie Leitungstreiber und Stromquelle minimal gehalten werden können. Eine weitere Maßnahme kann darin bestehen, dass die Kontaktplatte mit dem Leitungstreiber und der Stromquelle in einem abgeschirmten Gehäuse integriert sind.

Nach einer weiteren bevorzugten Ausführungsform ist eine Schaltung zur aktiven Kompensation der Streukapazität zwischen Kontaktplatte und Bezugspunkt vorgesehen. Auf diese Weise können in gewissen Grenzen Leitungslängen zwischen der Kontaktplatte sowie der Stromquelle und dem Leitungstreiber zugelassen werden, wobei die dadurch entstehenden Streukapazitäten durch die Kompensationsschaltung aufgehoben werden können.

Nach einer weiteren bevorzugten Ausführungsform ist vorgesehen, dass die Stromquelle eine bipolare Stromquelle mit hohem Innenwiderstand ist, deren Ausgangstrom annähernd proportional zur Eingangsspannung ist. Bipolare Stromquellen haben den Vorteil eines besonders hohen Innenwiderstandes und sind somit für die vorliegende Anwendung besonders geeignet.

Nach einer weiteren bevorzugten Ausführungsform ist vorgesehen, dass die Stromquelle einen Wechselstrom mit Frequenzen im Bereich von 1 kHz bis 1 MHz und Amplituden im Bereich von 1 $\mu$A bis 10 mA liefert. In der elektrischen Impedanz Tomographie werden überwiegend Ströme mit diesen Kenndaten eingesetzt. Selbstverständlich ist die Erfindung aber nicht auf diese Kenndaten beschränkt. Es ist auch denkbar, verschiedene Stromquellen mit verschiedenen Kenndaten einzusetzen, die sich in ihren Signalbereichen ergänzen oder überlappen. Außerdem ist es denkbar, dass der Wechselstrom frequenzmoduliert und/oder amplitudenmoduliert wird.

Nach einer weiteren bevorzugten Ausführungsform ist vorgesehen, dass der Leitungstreiber aus einer Impedanzwandlerschaltung besteht. Impedanzwandlerschaltungen sind in vielfältiger Weise bekannt, die einen hohen Eingangswiderstand in einen niedrigen Leitungswiderstand transformieren.

Nach einer weiteren bevorzugten Ausführungsform ist vorgesehen, dass zwischen dem niederohmigen Leitungsausgang und dem Bezugspunkt ein Spannungsmessgerät geschaltet ist. Auf diese Weise können beispielsweise Messungen nach der elektrischen Impedanz Tomographie durchgeführt werden, indem ein Strom über die Elektrode eingeprägt und die resultierende Spannung an der Elektrode abgegriffen wird. Besonders vorteilhaft ist es in diesem Zusammenhang, wenn zwischen der Kontaktplatte und dem Bezugspunkt ein Schalter vorgesehen ist, über den die Kontaktplatte wahlweise auf das Potential des Bezugspunktes schaltbar ist. Auf diese Weise kann eine bestimmte Elektrode oder können mehrere Elektroden von der Messung ausgenommen werden. Gleichzeitig kann eine Kalibrierung der Spannungsmessung gegenüber dem Bezugspunkt vorgenommen werden.

Der erfindungsgemäße Elektrodengürtel zur Durchführung einer Messung nach der elektrischen Impedanz Tomographie besteht aus einer Vielzahl von erfindungsgemäßen Elektroden. Die Elektroden sind dabei vorzugsweise im annähernd gleichen Abstand zueinander angebracht. Eine besonders zuverlässige Messwerterfassung und -übertragung kann durch eine digitale Datenerfassungseinheit erreicht werden. Hierzu ist mindestens ein Analog-Digital-Wandler vorgesehen, der von einer oder von mehreren Elektroden die Spannung zwischen der jeweiligen Kontaktplatte und dem Bezugspunkt einliest und über eine Datenleitung an eine zentrale Recheneinheit weiterleitet.

Im folgenden wird die Erfindung anhand verschiedener Ausführungsbeispiele mit Bezug auf die beiliegenden Zeichnungen näher erläutert. Es zeigen:

Fig. 1: einen Schnitt durch einen Brustkorb mit einer Elektrodenkonfiguration nach dem Stand der Technik,

Fig. 2: ein Prinzipschaltbild einer erfindungsgemäßen Elektrode,

Fig. 3: eine schematische Darstellung der Reziprok-Technik,

Fig. 4: ein Schaltbild für eine bipolare Stromquelle,

Fig. 5: ein Schaltbild für einen Leitungstreiber,

Fig. 6: einen Schnitt durch eine erfindungsgemäße Elektrode gemäß dem Prinzipschaltbild nach Fig. 2,

Fig. 7: einen Schnitt durch einen erfindungsgemäßen Elektrodengürtel,

Fig. 8: einen Schnitt durch eine Datenerfassungseinheit des Elektrodengürtels nach Fig. 7,

Fig. 9: eine Explosionsansicht der in Fig. 8 dargestellten Datenerfassungseinheit,

Fig. 10: eine Schaltung zur niederimpedanten, symmetrischen Signalübertragung und Verarbeitung,

Fig. 11: eine Schaltung zur Kompensation des hochohmigen Innenwiderstandes der Wechselstrom-Quellimpedanz einer Wechselstromquelle und

Fig. 12: eine Schaltung zur Kompensation von Streukapazitäten am Elektroden-Anschlusspunkt der Elektrode.

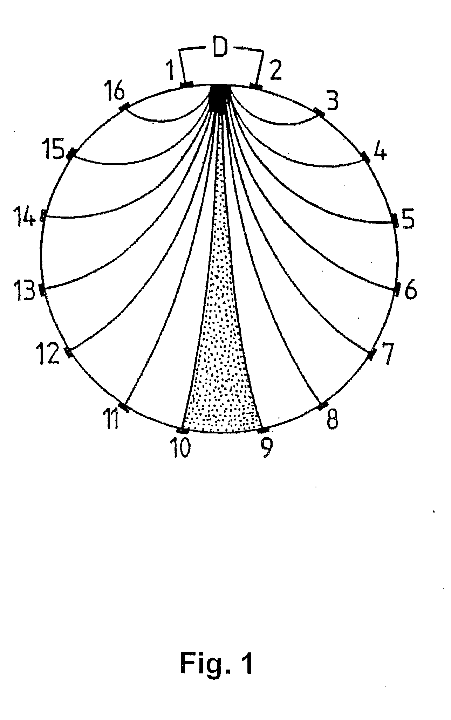

Fig. 1 zeigt einen Schnitt durch einen Brustkorb mit einer Elektrodenkonfiguration nach dem Stand der Technik (US 5,626,146). Um den Brustkorb herum sind 16 Elektroden im annähernd gleichen Abstand angeordnet. Zwischen den Elektroden 1 und 2 wird ein Strom eingeprägt. Die gekrümmten Linien sind dann Linien gleichen Potentials mit Bezug auf die als Dipol wirkenden Elektroden 1 und 2. Entlang dieser Linien können die an den Elektroden 3 bis 16 gemessenen Spannungspotentiale zurückverfolgt werden, um den Widerstandswert auf Punkten dieser Linien festzustellen. Nachdem für alle möglichen Dipole die jeweiligen Messungen durchgeführt wurden, können die aus jeder Messung ermittelten Impedanzwerte entsprechend überlagert werden. Dieses Verfahren der Rückverfolgungstechnik wurde hier als Beispiel gewählt und ist selbstverständlich nur eines neben vielen möglichen Verfahren der Bildrekonstruktion nach dem Prinzip der elektrischen Impedanz Tomographie.

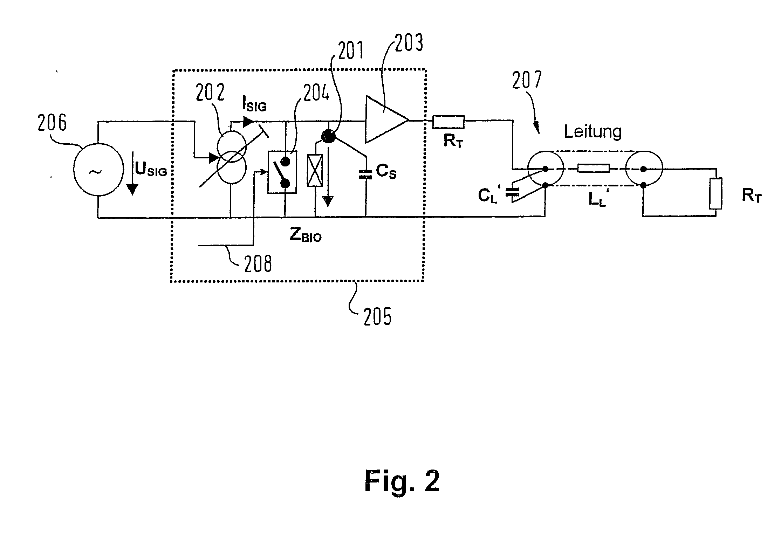

Fig. 2 zeigt ein Prinzipschaltbild einer erfindungsgemäßen Elektrode. Ihrem grundsätzlichen Aufbau nach besteht die Elektrode aus einer Kontaktplatte 201, einer Stromquelle 202 und einem Leitungstreiber 203. Die Kontaktplatte 201 ist zum einen mit dem Stromausgang der Stromquelle 202 und zum anderen mit dem hochohmigen Eingang des Leitungstreibers 203 verbunden. Die Stromquelle 202 ist mit ihrem anderen Pol mit einem Bezugspotential verbunden, wobei der Strom $I_{SIG}$ der Stromquelle annähernd proportional zu der von einer Spannungsquelle 206 gelieferten Eingangsspannung $U_{SIG}$ ist. Die Kontaktplatte 201 liegt auf der Haut des Patienten auf, wobei die Impedanz $Z_{BIO}$ zwischen Elektrode und Bezugspotential gemessen wird. Ziel der gesamten Schaltung ist es, die störenden Lastimpedanzen verursacht durch Streukapazitäten $C_S$ und ohmsche Nebenschlüsse zwischen der Kontaktplatte 201 und Bezugspotential möglichst gering zu halten. Zusätzlich ist zwischen Kontaktplatte und Bezugspotential ein steuerbarer Schalter 204 vorgesehen, der über die Steuerleitung 208 steuerbar ist und mit dem die Kontaktplatte 201 mit dem Bezugspotential verbunden werden kann. Auf diese Weise können einzelne Elektroden von der Messung ausgenommen werden oder aber eine Kalibrierung der Impedanzmessung vorgenommen werden. Am Ausgang des Leitungstreibers 203 wird die zwischen Elektrode und Bezugspotential anliegende Spannung gemessen. Gegebenenfalls wird die Messung über eine Leitung 207 durchgeführt, die in Fig. 2 mit ihrem entsprechenden Ersatzschaltbild dargestellt ist. Der gestrichelte Teil 205 der Schaltung ist in der Regel auf der Kontaktplatte 201 untergebracht, während die Spannungsquelle 206 beziehungsweise die Leitung 207 von der Kontaktplatte 201 weiter entfernt liegen können, ohne nennenswerte Störungen oder Messfehler zu verursachen.

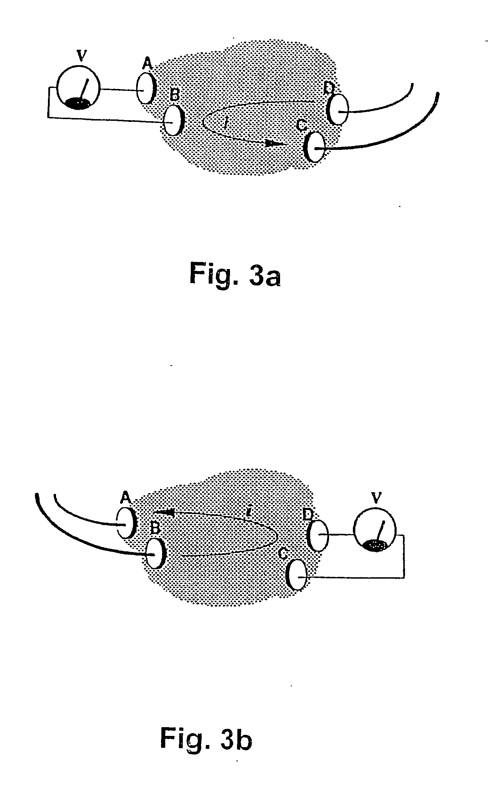

Fig. 3 zeigt eine schematische Darstellung der Reziprok-Technik. In Fig. 3a wird ein Strom über die Elektroden C und D eingeprägt und die resultierende Spannung an den Elektroden A und B gemessen. Umgekehrt wird in Fig. 3b über die Elektroden A und B ein entsprechender Strom eingeprägt und an den Elektroden C und D die resultierende Spannung gemessen. Es lässt sich zeigen, dass die jeweils gemessenen Spannungen aus der Fig. 3a und der Fig. 3b gleich sein müssen. Diese sogenannte Reziprok-Technik kann beispielsweise dazu verwendet werden, um die Kontakte an der Elektroden zu der Haut des Patienten auf einfache Weise zu überprüfen. Von den in Fig. 1 dargestellten Elektroden können dabei die jeweils gewünschten Elektroden durch Ansteuerung des Schalters 204 gemäß Fig. 2 ausgewählt werden.

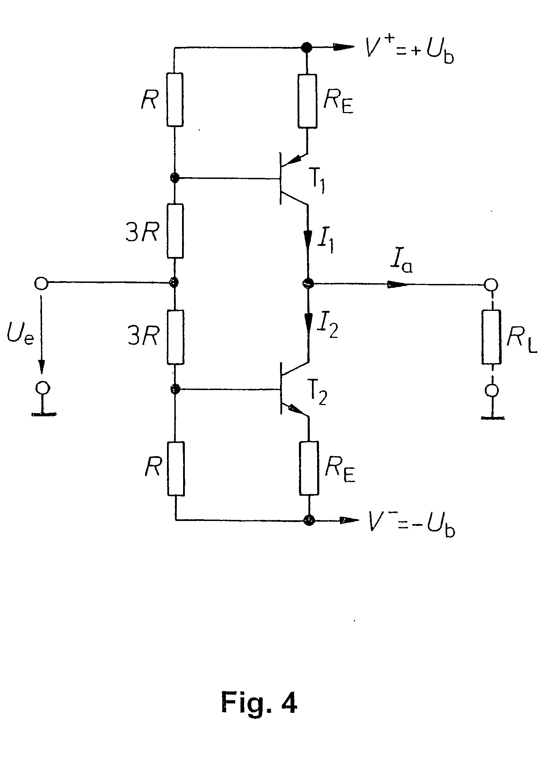

Fig. 4 zeigt ein Schaltbild für eine bipolare Stromquelle, die beispielsweise als Stromquelle 202 gemäß Fig. 2 verwendet werden kann. Wenn $U_e = 0$ ist, sind die beiden Ströme $I_1$ und $I_2$ gleich groß und der Ausgangsstrom ist Null. Legt man eine positive Eingangsspannung an, erhöht sich $I_2$ und $I_1$ nimmt ab, so dass ein negativer Ausgangsstrom fließt. Bei negativen Eingangsspannungen verhält sich die Schaltung entsprechend umgekehrt.

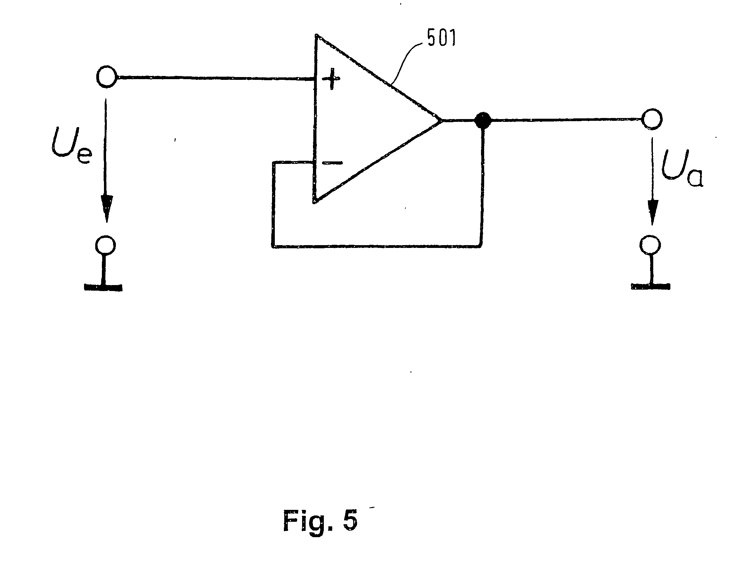

Fig. 5 zeigt ein Schaltbild für einen Leitungstreiber, der beispielsweise als Leitungstreiber 203 gemäß Fig. 2 eingesetzt werden kann. Der Operationsverstärker 501 ist demnach als Spannungsfolger beschaltet, so dass sich zwischen Ausgangs- und Eingangsspannung das Übersetzungsverhältnis 1 ergibt. Ein derartiger Spannungsfolger besitzt einen hohen Eingangswiderstand bei einem niedrigen Ausgangswiderstand, wobei der Offset zwischen Eingangs- und Ausgangsspannung nur wenige mV beträgt.

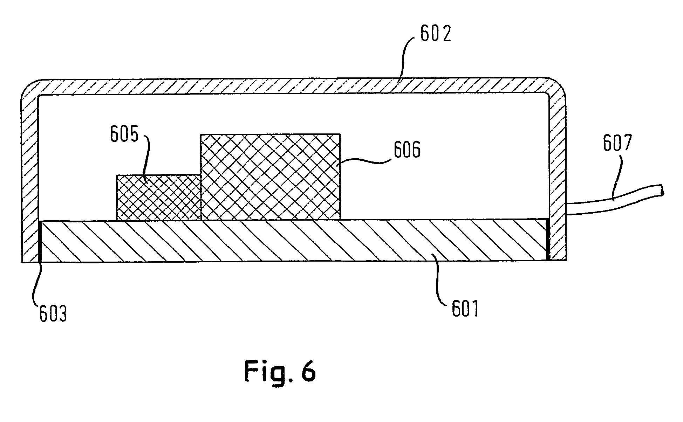

Fig. 6 zeigt einen Schnitt durch eine erfindungsgemäße Elektrode gemäß dem Prinzipschaltbild nach Fig. 2. Elektrisch gesehen entspricht der hier dargestellte Teil dem gestrichelten Schaltungsteil 205 gemäß Fig. 2. Die Kontaktplatte 601 wird von dem Gehäuse 602 umgeben, das eine abschirmende Wirkung haben kann und auf Bezugspotential gelegt werden kann. Für diesen Fall ist zwischen der Kontaktplatte 601 und dem Gehäuse 602 eine Isolierung 603 eingebracht. Auf der Kontaktplatte befindet sich eine integrierte Schaltung, die durch die Bausteine 605, 606 dargestellt ist und in der der Schaltungsteil 205 gemäß Fig. 2 realisiert ist. Gegebenenfalls ist der gesamte Schaltungsteil bereits in einem integrierten Schaltkreis untergebracht. Denkbar ist es auch, dass die Kontaktplatte 601 ebenfalls in diesem integrierten Schaltkreis auf der Unterseite untergebracht ist. Die elektrischen Zu- und Ableitungen werden in dem Kabel 607 geführt.

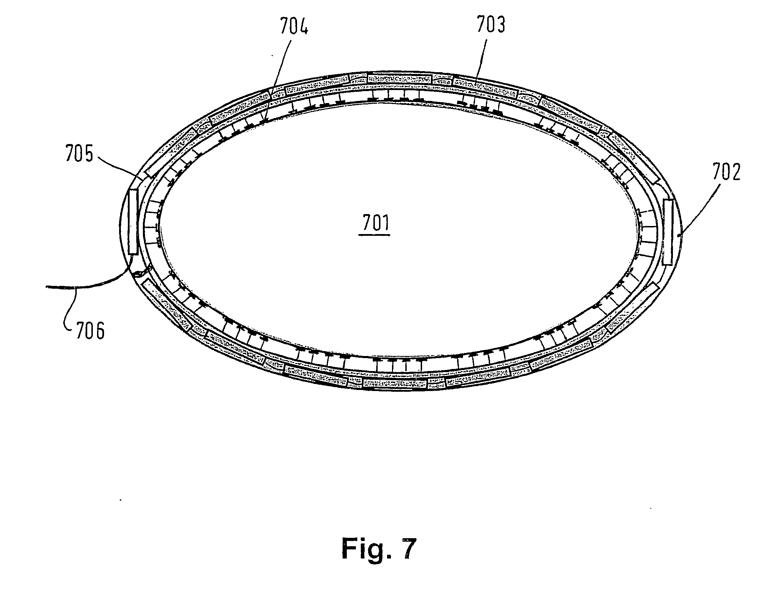

Fig. 7 zeigt einen Schnitt durch einen erfindungsgemäßen Elektrodengürtel. Der Elektrodengürtel 702 ist um den Brustkorb 701 des Patienten gelegt. An der Innenseite sind eine Vielzahl von Elektroden 704 angebracht, wobei jeweils vier Elektroden an einer Datenerfassungseinheit 703 angeschlossen sind. Die Datenerfassungseinheiten 703 sind durch Verbindungsleitungen 705 miteinander verbunden, wobei die elektrischen Zu- und Ableitungen in dem Kabel 706 geführt werden.

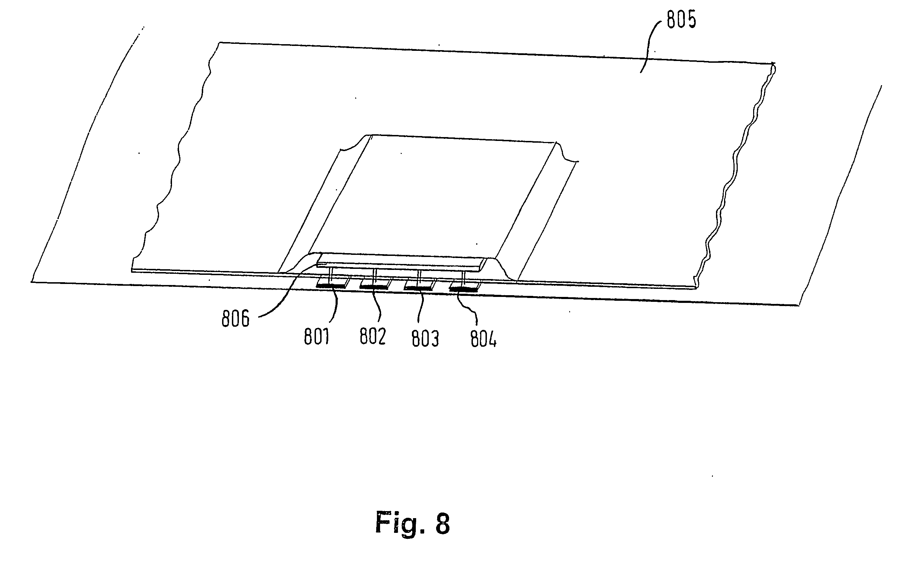

Fig. 8 zeigt einen Schnitt durch eine Datenerfassungseinheit des Elektrodengürtels nach Fig. 7. Vier Elektroden 801, 802, 803 und 804 sind am unteren Rand des Gürtelmaterials 805 befestigt und mit der Auswerteelektronik 806 elektrisch verbunden. Bei der Auswerteelektronik bieten sich verschiedene Formen der Datenerfassung an:

Parallelverfahren: Jeder Kanal wird synchron mittels eines eigenen Analog-Digitalwandlers (ADC) digitalisiert. Das jeweilige Wandlungsergebnis steht parallel in je einem lokalen Digitalspeicher zum Transfer über eine digitale Datenbusstruktur für die weitere Signalverarbeitung wahlfrei zur Verfügung.

Analog-Multiplex: Im Zeit-Multiplexverfahren werden nach festgelegtem Schema alle Messkanäle einzeln per Multiplexer an einen einzigen leistungsfähigen ADC geschaltet und durch diesen digitalisiert. Ein externer Digitalspeicher nimmt dabei, nach Transfer über eine Datenschnittstelle, alle aufeinanderfolgenden Daten in einer Wertetabelle auf.

Integrierte Datenerfassung: Ein monolithisch integriertes Datenerfassungssystem, bestehend aus auf einem Mikrochip integriertem Eingangskanal-Multiplexer, Verstärker mit wählbarem Verstärkungsfaktor, nachgeschaltetem Analog-Digital-Wandler, lokalem Speicher für die Aufnahme einer größeren Anzahl von digitalisierten Messwerten, sowie einer autarken Ablaufsteuerung, angeschlossen an eine Kommunikationsschnittstelle oder den Datenbus eines Wirtsrechners, erfasst sequentiell eigenständig eine Anzahl von Eingangskanälen und speichert die digitalisierten Messwerte im lokalen Speicher. Bei erreichen eines bestimmten Füllungsgrades des Speichers wird der Wirtsrechner zur schnellen Übernahme der erfassten Daten veranlasst. Damit steht wieder Platz für weitere Daten des Digital-Analog-Wandlers im lokalen Speicher zur Verfügung. Das neben dem Patienten aufgestellte Rechnersystem wird durch ein solches teil-autonom arbeitendes Datenerfassungssystem wirkungsvoll entlastet.

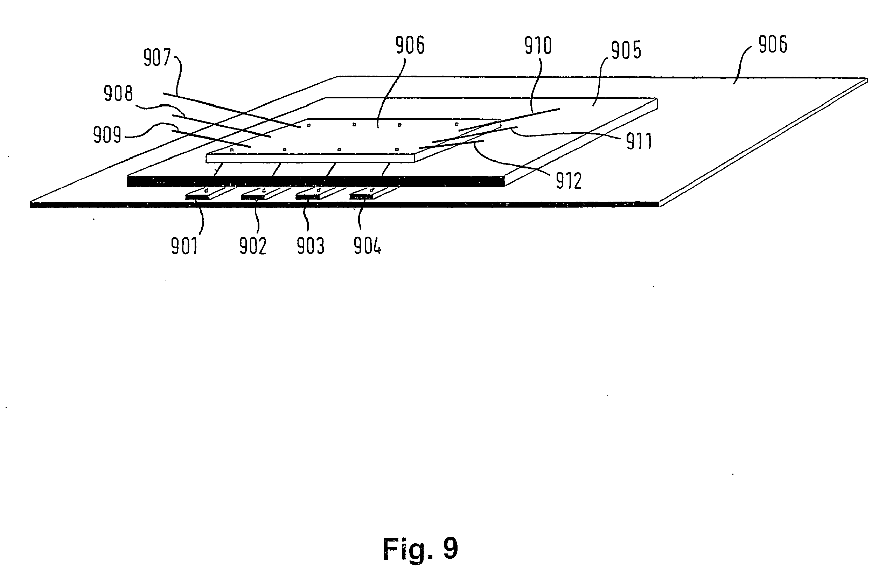

Fig. 9 zeigt eine Explosionsansicht der in Fig. 8 dargestellten Datenerfassungseinheit. Die Kontaktplatten 901, 902, 903 und 904 sind an dem Gürtelmaterial 905 befestigt und liegt auf der Haut 906 des Patienten auf. Auf der anderen Seite des Gürtelmaterials befindet sich die Datenerfassungseinheit 906 mit entsprechenden Durchkontaktierungen zu den Kontaktplatten 901, 902, 903 und 904. Die Leitungen 907 bis 912 stellen Kontaktleitungen zu den jeweils benachbarten Datenerfassungseinheiten dar (Signaleingang 907, Versorgungsspannungseingang 908, Steuerleitungen 909, Datenausgang 910, Versorgungsspannungsausgang 911 und Steuerleitungen 912).

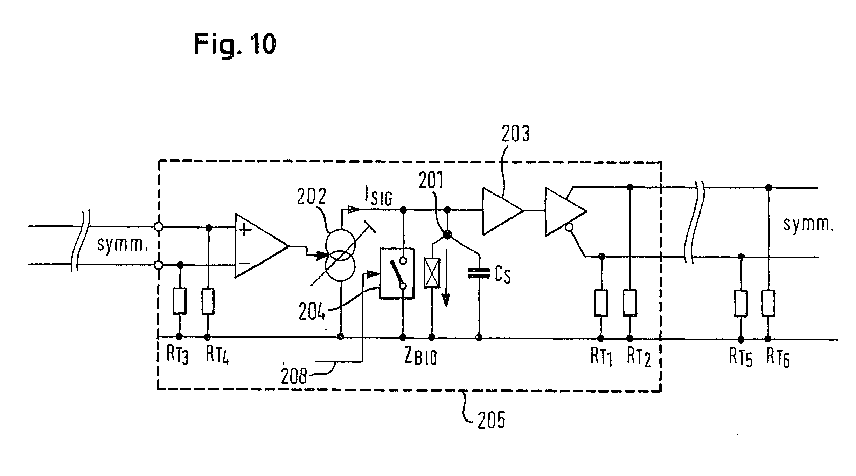

Eine Schlüsselgröße für den Einsatz in der elektrischen Impedanz-Tomographie ist ein möglichst großer Signal/Rauschabstand. Hierzu wird in dem Ausführungsbeispiel gemäß Fig. 10 eine möglichst niederimpedant auszuführende, symmetrische Signalübertragung vorgeschlagen. In der Konsequenz heißt dies die Erweiterung um entsprechende symmetrische Buffer am Elektrodenausgang und symmetrische Leitungsempfänger samt Abschlusswiderständen auf der Sendeseite.

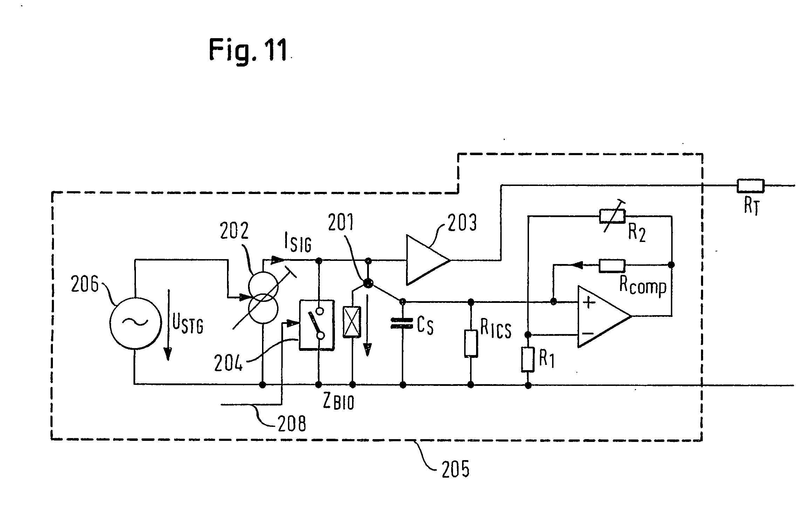

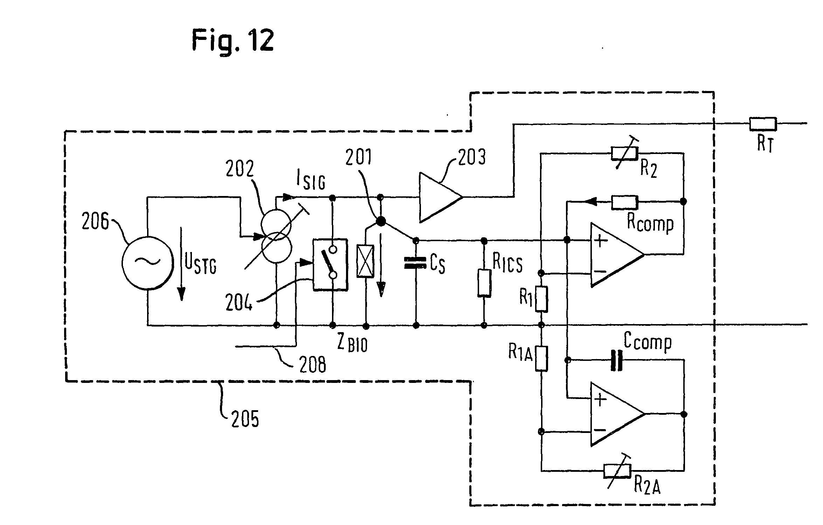

In den Ausführungsbeispielen gemäß Fig. 11 und Fig. 12 wird eine Kompensation bzw. Teilkompensation der Parasitärkapazitäten sowie der verbleibenden Wirk-Innenwiderstände der Stromquellen vorgeschlagen. Diese Kompensation bzw. Teilkompensation kann aktiv und/oder resistiv und/oder kapazitiv erfolgen.

1. Niederimpedante, symmetrische Signalübertragung und Verarbeitung:

Der Störabstand zu den Elektroden kann verbessert werden durch,

a) eine symmetrische Signalübertragung mit

b) niederimpedanten Abschlußimpedanzen ($R_{Ti}$ mit $R_{Ti} <= 300$ Ohm typ.) und

c) verbesserter Entkopplung der Stromversorgungs- und Multiplexerstrukturen

Eine entsprechende Schaltung zeigt Fig. 10:

Vor dem Steuereingang der Wechselstromquelle 202 wird ein für die symmetrische Signalübertragungstechnik geeigneter Leitungsempfänger (Differenzverstärker) hinzugefügt. - Der symmetrische Leitungsabschluß wird mit den niederimpedanten Abschlußimpedanzen ($R_{T3}$, $R_{T4}$) erreicht.

Entsprechend wird ein symmetrischer Leitungstreiber am Ausgang des Buffers 203 hinzugefügt. Mit den zugehörigen niederohmigen Abschlußwiderständen ($R_{T1}$, $R_{T2}$) bzw. ($P_{T5}$, $R_{T6}$) für die doppelte Terminierung an Anfang und Ende einer längeren Leitung wird ein insgesamt sehr günstiges und vorteilhaftes niederimpedantes Leitungssystem geschaffen, da Störeinflüsse die in das symmetrische Leitungssystem einkoppeln in den jeweiligen Leitungsempfängern weitgehend subtrahiert werden.

Weiterhin vorteilhaft werden die Zeitkonstanten der Schalttransienten, gebildet etwa durch geladene Eingangskapazitäten und die Eingangs- oder Ausgangsimpedanzen des Leitungssystem sehr günstig beeinflusst. Außerdem wird auch die Gefahr kapazitiver Störeinkopplungen mit nennenswerten Amplituden auf die sehr niederohmigen Leiter von außen noch erheblich erschwert.

Insgesamt ergibt sich, bei erhöhtem Leitungsaufwand, damit aber eine gleich in mehrfacher Hinsicht vorteilhafte Ergänzung die zu wesentlich günstigeren S/N-Werten führt.

2. Kompensation des hochohmigen Innenwiderstandes der

Wechselstrom-Quellimpedanz einer Wechselstromquelle.

Das in Fig. 11 skizzierte, zusätzlich anwendbare Kompensationsverfahren für eine mindestens teilweise mögliche Kompensation des hochohmigen reellen Quellwiderstandes ($R_{ICS}$) einer Wechselstromquelle als Anteil der komplexen Wechselstrom-Quellimpedanz erklärt sich in seiner Funktion wie folgt:

Der zusätzliche Operationsverstärker mit seinen Beschaltungselementen R1, R2 und $R_{COMP}$ stellen hierbei eine Kompensationsschaltung für den positiven Quellenwiderstand $R_{ICS}$ dar.

Der Operationsverstärker, der mit seinem nichtinvertierenden Eingang an den Knotenpunkt 201 geschaltet ist, kann unter einer gedachten Weglassung von $R_{COMP}$ zunächst als nichtinvertierende Grundschaltung verstanden werden, deren Spannungsverstärkung von diesem Eingang zum Ausgang von der Beschaltung durch die Widerstände R1 und R2 bestimmt wird gemäß U_AUS/U_EIN = 1+ R2/R1. Das Verhältnis von R2/R1 bestimmt also die Höhe der Ausgangsspannung U_AUS an dessen Ausgang. - Wählt man das Verhältnis zu R2/R1 = 1, so ergibt sich die Spannungsverstärkung U_AUS/U_EIN = +2.

Betrachtet man nun die Spannungen und Ströme um den Quellwiderstand $R_{ICS}$, so ergibt sich ein positiver Teilstrom durch $R_{ICS}$, gemäß I = U/R. Ein weiterer reeller Stromanteil könnte sich ggfs. auch durch die ebenfalls parallel geschaltete Bio-Impedanz $Z_{BIO}$ nach den Regeln der Stromteilung ergeben. $Z_{BIO}$ sei hier aber zunächst als reiner Blindwiderstand angenommen.

Wird nun der vom Ausgang des Operationsverstärkers zu Knoten 201 geschaltete Widerstand $R_{COMP}$ betrachtet, so ergibt sich ein weiterer Strompfad für jede Spannungsdifferenz zwischen dem Knoten 201 und der Ausgangsspannung des Operationsverstärkers, die über das Widerstandsverhältnis R2/R1 bestimmt ist.

Für die Annahme einer Spannungsverstärkung des OPAMPs von U_AUS/U_EIN = +2 wird die Ausgangsspannung also genau den doppelten Betrag der Spannung an Knoten 201 betragen. Wird der Widerstandswert von $R_{COMP}$ identisch wie $R_{ICS}$ gewählt, so kompensieren sich die via $R_{COMP}$ zum Knoten 201 zufließenden und via $R_{ICS}$ abfließenden reellen Teilströme gerade aufgrund gleicher Beträge aber entgegengesetzter Vorzeichen: Die OPAMP-Schaltung um $R_{COMP}$ lässt diesen negativ erscheinen, so dass gilt $R_{ICS}$ - $R_{COMP}$ = 0 oder, ausgedrückt als Kehrwert der Leitwerte für die resultierende Parallelschaltung Rp

$$\overline{1/(1/R_{ICS} - 1/R_{COMP}) = Rp.}$$

Für die Identität $R_{ICS}$ = $R_{COMP}$ ergibt sich eine 0 im Nenner dieses Bruchs - eine Polstelle für den Widerstandswert des Ausdrucks.

Durch Wahl von $R_{COMP}$ oder des Widerstandsverhältnisses R2/R1 für die Wahl der Spannungsverstärkung kann sowohl über- als auch unterkompensiert werden. - Der erste Fall führt zu Instabilität und einem unbrauchbaren Schwingen der Schaltung.

Die Unterkompensation aber erlaubt die sehr vorteilhafte, graduell einstellbare künstliche Erhöhung der Quellimpedanz der Stromquelle, mit dem Vorzug einer qualitativ erheblich gesteigerten Präzision für entsprechende Spannungsmessungen via Buffer 203.

Das Verfahren erlaubt darüber hinaus auch die Kompensation weiteres parallel geschalteter reeller Widerstände und ist daher universell verwenbar.

3. Kompensation von Streukapazitäten am Elektroden-Anschlußpunkt der Elektrode

Fig. 12 zeigt das auf eine kapazitive Kompensation erweiterte Verfahren auf. - Hierbei können wiederum alle parallel geschalteten Kapazitäten zusammengefasst werden zu einer, die dann stellvertretend zu einem großen Teil kompensiert werden kann: $C_{COMP}$ = ($C_{ICS}$ + $C_S$).

Die Schaltung gemäß Fig. 12 unterscheidet sich gegenüber der Schaltung gemäß Fig. 11 dadurch, dass nun anstelle eines reellen Quellwiderstandes $R_{ICS}$ eine zu kompensierende Kapazität ($C_{ICS}$ + $C_S$) mit ihrem Blindwiderstand

$X_{ICS}$ = 1 /2*PI*f*($C_{ICS}$ + $C_S$) und statt des resistiven Elementes $R_{COMP}$ nun ein Blindwiderstand

$X_{COMP}$ = 1 /2*PI*f*$C_{COMP}$ eingesetzt wird.

Dabei stellt die Variable f jeweils die Betriebsfrequenz der Schaltung dar.

Mit dem dargestellten Doppelansatz für die resistive und kapazitive Teilkompensation sind die parasi-

täreren Einflüsse auch einer Active Probe also noch erheblich weiter zu reduzieren und so der nutzbare Frequenzbereich von der ursprünglich angedachten Betriebsfrequenz von 10 kHz oder 25 kHz auch über 100 kHz hinaus zu erweitern.

**Patentansprüche**

1. Elektrode für biomedizinische Messungen,
   mit einer Kontaktplatte (201), und
   mit einem in räumlicher Nähe zur Kontaktplatte angeordneten Leitungstreiber (203) der einen hochohmigen Signaleingang und einen niederohmigen Leitungsausgang aufweist,
   **gekennzeichnet durch**
   eine in räumlicher Nähe zur Kontaktplatte angeordnete Stromquelle (202), die einen Stromausgang sowie einen Bezugspunkt aufweist,
   wobei die Kontaktplatte mit dem hochohmigen Signaleingang des Leitungstreibers und mit dem Stromausgang der Stromquelle elektrisch verbunden ist und
   wobei zwischen dem niederohmigen Leitungsausgang und dem Bezugspunkt eine Messspannung abgreifbar ist.

2. Elektrode nach Anspruch 1, **dadurch gekennzeichnet, dass** die elektrischen Verbindungen zwischen der Kontaktplatte, der Stromquelle sowie dem Leitungstreiber derart ausgestaltet sind, dass sich eine möglichst niedrige eine möglichst niedrige Streukapazität ($C_3$) zwischen Kontaktplatte und Bezugspunkt ergibt.

3. Elektrode nach einem der Ansprüche 1 - 2, **dadurch gekennzeichnet, dass** der Leitungstreiber und die Stromquelle sich als integrierte Schaltung auf der Kontaktplatte befinden.

4. Elektrode nach einem der Ansprüche 1 - 3, **dadurch gekennzeichnet, dass** die Kontaktplatte (601) mit dem Leitungstreiber und der Stromquelle in einem abgeschirmten Gehäuse (602) integriert sind.

5. Elektrode nach einem der Ansprüche 1 - 4, **dadurch gekennzeichnet, dass** eine Schaltung zur aktiven Kompensation der Streukapazität zwischen Kontaktplatte und Bezugspunkt vorgesehen ist.

6. Elektrode nach einem der Ansprüche 1 - 5, **dadurch gekennzeichnet, dass** die Stromquelle eine bipolare Stromquelle mit hohem Innenwiderstand ist, deren Ausgangstrom annähernd proportional zur Eingangsspannung ist.

7. Elektrode nach einem der Ansprüche 1 - 6, **dadurch gekennzeichnet, dass** die Stromquelle einen Wechselstrom mit Frequenzen im Bereich von 1 kHz bis 1 MHz und Amplituden im Bereich von 1 µA bis 10 mA liefert.

8. Elektrode nach Anspruch 7, **dadurch gekennzeichnet, dass** der Wechselstrom frequenzmoduliert und/oder amplitudenmoduliert ist.

9. Elektrode nach einem der Ansprüche 1 - 8, **dadurch gekennzeichnet, dass** der Leitungstreiber aus einer Impedanzwandlerschaltung besteht.

10. Elektrode nach Anspruch 9, **dadurch gekennzeichnet, dass** zwischen dem niederohmigen Leitungsausgang und dem Bezugspunkt ein Spannungsmessgerät geschaltet ist.

11. Elektrode nach einem der Ansprüche 1 - 10, **dadurch gekennzeichnet, dass** zwischen der Kontaktplatte und Bezugspunkt ein Schalter (204) vorgesehen ist, über den die Kontaktplatte wahlweise auf das Potential des Bezugspunktes schaltbar ist.

12. Elektrodengürtel zur Durchführung einer Messung nach der elektrischen Impedanz Tomographie mit einer Vielzahl von Elektroden nach einem der Ansprüche 1 - 11.

13. Elektrodengürtel nach Anspruch 12, **dadurch gekennzeichnet, dass** die Elektroden im annähernd gleichen Abstand zueinander angebracht sind.

14. Elektrodengürtel nach einem der. Ansprüche 12 - 13, **dadurch gekennzeichnet, dass** Datenerfassungseinheiten (703) mit mindestens einem Analog-Digital-Wandler vorgesehen sind, die von einer oder von mehreren Elektroden die Spannung zwischen der jeweiligen Kontaktplatte und dem Bezugspunkt über den Analog-Digital-Wandler einlesen und über eine Datenleitung an eine zentrale Recheneinheit weiterleiten.

**Claims**

1. Electrode for biomedical measurements,
   with a contact plate (201), and
   with a line driver (203) which has a high-impedance signal input and a low-impedance line output and is arranged in the vicinity of the contact plate,
   **characterized by**
   a current source (202) which has a current output and a reference point and is arranged in the vicinity of the contact plate,
   wherein the contact plate is electrically connected to the high-impedance signal input of the line driver and to the current output of the current source and
   wherein a measuring circuit voltage can be picked up between the low-impedance line output and the reference point.

2. Electrode according to claim 1, **characterized in that** the electrical connections between the contact plate, the current source and the line driver are designed in such a way that a stray capacitance ($C_3$) which is as low as possible is produced between the contact plate and reference point.

3. Electrode according to any one of the claims 1 - 2, **characterized in that** the line driver and the current source are located as integrated circuit on the contact plate.

4. Electrode according to any one of claims 1 - 3, **characterized in that** the contact plate (601) with the line driver and the current source are integrated in a shielded housing (602).

5. Electrode according to any one of claims 1 - 4, **characterized in that** a circuit is provided for active compensation of the stray capacitance between the contact plate and reference point.

6. Electrode according to any one of claims 1 - 5, **characterized in that** the current source is a bipolar current source with high internal resistance, the output current of which is virtually proportional to the input voltage.

7. Electrode according to any one of claims 1 - 6, **characterized in that** the current source supplies an alternating current with frequencies in the range from 1 kHz to 1 MHz and amplitudes in the range from 1 μA to 10 mA.

8. Electrode according to claim 7, **characterized in that** the alternating current is frequency-modulated and/or amplitude-modulated.

9. Electrode according to any one of claims 1 - 8, **characterized in that** the line driver consists of an impedance converter circuit.

10. Electrode according to claim 9, **characterized in that** a voltage measuring apparatus is connected between the low-impedance line output and the reference point.

11. Electrode according to any one of claims 1 - 10, **characterized in that** a switch (204), via which the contact plate can be optionally switched to the potential of the reference point, is provided between the contact plate and reference point.

12. Electrode belt for carrying out a measurement by electrical impedance tomography with a plurality of electrodes according to any one of claims 1 - 11.

13. Electrode belt according to claim 12, **characterized in that** the electrodes are provided with virtually equal spacing from one another.

14. Electrode belt according to any one of the claims 12 - 13, **characterized in that** data acquisition units (703) with at least one analogue-to-digital converter are provided which from one or more electrodes read in the voltage between the respective contact plate and the reference point via the analogue-to-digital converter and pass it to a central processor via a data line.

## Revendications

1. Electrode conçue pour des mesures biomédicales, avec une plaque de contact (201), et avec un amplificateur de ligne (203), disposé à proximité spatiale de la plaque de contact et présentant une entrée de signal fortement ohmique et une sortie de ligne faiblement ohmique, **caractérisée par** une source de courant (202), disposée à proximité spatiale de la plaque de contact et présentant une sortie de courant ainsi qu'un point de référence, la plaque de contact étant relié électriquement à l'entrée de signal, fortement ohmique, de l'amplificateur de ligne et à la sortie de courant de la source de courant, et une tension de mesure étant susceptible de ligne d'être prélevée, entre la sortie de ligne, faiblement ohmique, et le point de référence.

2. Electrode selon la revendication 1, **caractérisée en ce que** les liaisons électriques entre la plaque de contact, la source de courant ainsi que l'amplificateur de ligne sont réalisées de manière qu'il y ait une capacité parasite ($C'_3$) aussi faible que possible entre la plaque de contact et le point de référence.

3. Electrode selon l'une des revendications 1 à 2, **caractérisée en ce que** l'amplificateur de ligne et la source de courant se trouvent, sous forme de circuit intégré, sur la plaque de contact.

4. Electrode selon l'une des revendications 1 à 3, **caractérisée en ce que** la plaque de contact (601), avec l'amplificateur de ligne et la source de courant, sont intégrés dans un boîtier (602) écranté.

5. Electrode selon l'une des revendications 1 à 4, **caractérisée en ce qu'**un circuit, de compensation active de la capacité parasite, est prévu entre la plaque de contact et le point de référence.

**6.** Electrode selon l'une des revendications 1 à 5, **caractérisée en ce que** la source de courant est une source de courant bipolaire ayant une haute résistance intérieure, dont le courant de sortie est à peu près proportionnel à la tension d'entrée.

**7.** Electrode selon l'une des revendications 1 à 6, **caractérisée en ce que** la source de courant fournit un courant alternatif ayant des fréquences dans la plage de 1 kHz à 1 MHz et des amplitudes dans la plage de 1 $\mu$A à 10 $\mu$A.

**8.** Electrode selon la revendication 7, **caractérisée en ce que** le courant alternatif est modulé en fréquence ct/ou modulé en amplitude.

**9.** Electrode selon l'une des revendications 1 à 8, **caractérisée en ce que** l'amplificateur de ligne est composé d'un circuit convertisseur d'impédance.

**10.** Electrode selon la revendication 9, **caractérisée en ce qu'**un appareil de mesure de tension est branché entre la sortie de ligne, faiblement ohmique, et le point de référence.

**11.** Electrode selon l'une des revendications 1 à 10, **caractérisée en ce qu'**entre la plaque de contact et le point de référence est prévu un interrupteur (204), par l'intermédiaire duquel la plaque de contact est susceptible d'être branchée, au choix, au potentiel du point de référence.

**12.** Ceinture à électrodes, pour effectuer une mesure selon la tomographie à impédance électrique, avec une pluralité d'électrodes selon l'une des revendications 1 à 11.

**13.** Ceinture à électrodes selon la revendication 12, **caractérisée en ce que** les électrodes sont montées avec à peu près le même espacement mutuel entre elles.

**14.** Ceinture à électrodes selon l'une des revendications 12 à 13, **caractérisée en ce que** des unités de détection de données (703), comprenant au moins un convertisseur analogique-numérique, sont prévues, lisant, à partir d'une ou plusieurs électrodes, la tension entre la plaque de contact respective et le point de référence, et la retransmettant à une unité de calcul centrale, par l'intermédiaire d'une ligne de données.

Fig. 1

**Fig. 2**

EP 1 455 647 B1

Fig. 3a

Fig. 3b

**Fig. 4**

**Fig. 5**

EP 1 455 647 B1

Fig. 6

**Fig. 7**

Fig. 8

Fig. 9

# Fig. 10

EP 1 455 647 B1

# Fig. 11

EP 1 455 647 B1

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0033733 A
- US 5544662 A
- US 5626146 A

**In der Beschreibung aufgeführte Nicht-Patentliteratur**

- **J.D. BRONZINO.** The Biomedical Engineering Handbook. CRC Press, 1995, 745-757